# EUROPEAN PATENT APPLICATION

(11) **EP 4 847 888 A1**
(43) Date of publication of application: **02.12.2026**
(21) Application number: 26179862.3
(22) Date of filing: 20.05.2026
(51) Int. Cl.: G01F 23/24, C12M 1/34, G01F 23/26

(54) **MULTIPLE-LEVEL LIQUID AND FOAM DETECTOR CONDUCTIVE SENSOR FOR BIOPROCESS EQUIPMENT**

(30) Priority: 21.05.2025 US 202563809482 P
(71) Applicant: Univercells Technologies SA, 1400 Nivelles (BE)
(72) Inventor: De Viron, Laetitia, 1400 Nivelles (BE); Drugmand, Jean-Christophe, 1400 Nivelles (BE)
(74) Representative: Brantsandpatents bv

(57) **Abstract**

Embodiments herein relate to sensors and detection systems for monitoring liquid and foam levels within bioprocess equipment. In an embodiment, a multi-level liquid and foam sensor for bioprocess equipment is included having a probe shaft, a plurality of conductive probe wires arranged at different vertical levels along a height of the probe shaft, and a controller in electrical communication with the plurality of conductive probe wires. The controller can be configured to sense electrical properties of the plurality of conductive probe wires and estimate a liquid level within the bioprocess equipment based on the sensed electrical properties. Other embodiments are also included herein.

## Description

### Field

Embodiments herein relate to sensors and detection systems for monitoring liquid and foam levels within bioprocess equipment, particularly for disposable bioprocess equipment with applications in biotechnological and pharmaceutical processes.

### Background

Bioreactors, as one example of bioprocess equipment, are systems used in the biotechnological and pharmaceutical industries, used for cultivating microorganisms, cells, or biochemical substances under controlled conditions. The monitoring of liquid levels within these reactors or containers in biotech/pharma applications can aid in maintaining optimal conditions for biological reactions and product yield.

Traditional methods for monitoring liquid levels in bioreactors and other pieces of bioprocess equipment have relied on various types of sensors, including optical, ultrasonic, and mechanical float sensors. However, these methods often face challenges related to accuracy, response time, and maintenance, especially in the dense and complex media typically found in bioprocess equipment. Additional challenges relate to the need to maintain aseptic conditions within bioprocess equipment and that measuring equipment should not interact with or contaminate the liquid within bioprocess equipment.

### Summary

Embodiments herein relate to sensors and detection systems for monitoring liquid and foam levels within bioprocess equipment, particularly for disposable bioprocess equipment with applications in biotechnological and pharmaceutical processes. In a first aspect, a multi-level liquid and foam sensor for bioprocess equipment can be included. The sensor can include a probe shaft and a plurality of conductive probe wires, wherein the plurality of conductive probe wires can be arranged at different vertical levels along a height of the probe shaft. The sensor can further include a controller in electrical communication with the plurality of conductive probe wires. The controller can be configured to sense electrical properties of the plurality of conductive probe wires and estimate a liquid level within a piece of bioprocess equipment based on the sensed electrical properties.

In a second aspect, in addition to one or more of the preceding or following aspects, or in the alternative to some aspects, the controller can be configured to detect a height of liquid in the vessel using the sensed electrical properties of at least two conductive probe wires.

In a third aspect, in addition to one or more of the preceding or following aspects, or in the alternative to some aspects, the controller can be configured to detect the presence of foam above the liquid level using the sensed electrical properties.

In a fourth aspect, in addition to one or more of the preceding or following aspects, or in the alternative to some aspects, the controller can be configured to detect a height of foam above the liquid level using the sensed electrical properties of conductive probe wires.

In a fifth aspect, in addition to one or more of the preceding or following aspects, or in the alternative to some aspects, the conductive probe wires can include exposed end portions.

In a sixth aspect, in addition to one or more of the preceding or following aspects, or in the alternative to some aspects, the plurality of conductive probe wires can include an insulator coated over outside surfaces of the plurality of conductive probe wires other than at the exposed end portions thereof.

In a seventh aspect, in addition to one or more of the preceding or following aspects, or in the alternative to some aspects, the exposed end portions can be disposed outside of the probe shaft.

In an eighth aspect, in addition to one or more of the preceding or following aspects, or in the alternative to some aspects, the exposed end portions fit within one or more grooves in an exterior surface of the probe shaft.

In a ninth aspect, in addition to one or more of the preceding or following aspects, or in the alternative to some aspects, the length of the exposed end portions disposed outside of the probe shaft can be at least 0.5 centimeters in length.

In a tenth aspect, in addition to one or more of the preceding or following aspects, or in the alternative to some aspects, the length of the exposed end portions disposed outside of the probe shaft can be 0.5 to 3 centimeters in length.

In an eleventh aspect, in addition to one or more of the preceding or following aspects, or in the alternative to some aspects, the exposed end portions can be wrapped around an outside surface of the probe shaft.

In a twelfth aspect, in addition to one or more of the preceding or following aspects, or in the alternative to some aspects, the exposed end portions of the plurality of conductive probe wires can be disposed in a spiral configuration along the height of the probe shaft.

In a thirteenth aspect, in addition to one or more of the preceding or following aspects, or in the alternative to some aspects, the exposed end portions of the plurality of conductive probe wires can be disposed in a straight line configuration along the height of the probe shaft.

In a fourteenth aspect, in addition to one or more of the preceding or following aspects, or in the alternative to some aspects, the exposed end portions can be at least partially wrapped around an outside surface of the probe shaft.

In a fifteenth aspect, in addition to one or more of the preceding or following aspects, or in the alternative to some aspects, the exposed end portions can be completely wrapped around an outside surface of the probe shaft.

In a sixteenth aspect, in addition to one or more of the preceding or following aspects, or in the alternative to some aspects, the sensed electrical properties include at least one of potential difference, conductance, resistance, capacitance, and impedance.

In a seventeenth aspect, in addition to one or more of the preceding or following aspects, or in the alternative to some aspects, the plurality of conductive probe wires includes from 2 to 15 conductive probe wires.

In an eighteenth aspect, in addition to one or more of the preceding or following aspects, or in the alternative to some aspects, at least two of the plurality of conductive probe wires can be disposed adjacent a bottom of the probe shaft and others can be spaced apart vertically along a vertical axis of the probe shaft.

In a nineteenth aspect, in addition to one or more of the preceding or following aspects, or in the alternative to some aspects, conductivity of a liquid within the piece of bioprocess equipment can be determined based on electrical properties of a circuit including the at least two conductive probe wires disposed adjacent a bottom of the probe shaft.

In a twentieth aspect, in addition to one or more of the preceding or following aspects, or in the alternative to some aspects, the controller can be further configured to adjust a sensitivity of foam detection based on the conductivity of the liquid within the piece of bioprocess equipment.

In a twenty-first aspect, in addition to one or more of the preceding or following aspects, or in the alternative to some aspects, the controller can be configured to calculate a liquid level based on differing electrical properties between two conductive probe wires.

In a twenty-second aspect, in addition to one or more of the preceding or following aspects, or in the alternative to some aspects, the controller can be configured to calculate a volume of liquid based on the liquid level and the geometry of the piece of bioprocess equipment.

In a twenty-third aspect, in addition to one or more of the preceding or following aspects, or in the alternative to some aspects, the controller can be configured to calculate a liquid level based on differing electrical properties between two adjacent conductive probe wires.

In a twenty-fourth aspect, in addition to one or more of the preceding or following aspects, or in the alternative to some aspects, the controller can be configured to detect the presence of foam above the liquid level based on differing electrical properties between two conductive probe wires.

In a twenty-fifth aspect, in addition to one or more of the preceding or following aspects, or in the alternative to some aspects, the controller can be configured to detect the height of foam above the liquid level based on differing electrical properties between two conductive probe wires.

In a twenty-sixth aspect, in addition to one or more of the preceding or following aspects, or in the alternative to some aspects, the controller can be configured to detect the type of foam based on differing electrical properties between two conductive probe wires.

In a twenty-seventh aspect, in addition to one or more of the preceding or following aspects, or in the alternative to some aspects, the sensor can further include a connection fitting, wherein the connection fitting can be disposed adjacent a top of the probe shaft and allows the fluid and foam sensor to engage with a standardized port in a housing of the piece of bioprocess equipment.

In a twenty-eighth aspect, in addition to one or more of the preceding or following aspects, or in the alternative to some aspects, the standardized port can include a PDG 13.5 port.

In a twenty-ninth aspect, in addition to one or more of the preceding or following aspects, or in the alternative to some aspects, the housing can include a non-conductive housing material.

In a thirtieth aspect, in addition to one or more of the preceding or following aspects, or in the alternative to some aspects, the sensor can further include a wireless transmitter, wherein the wireless transmitter can be configured to send data regarding the liquid level and foam presence to a remote processing unit.

In a thirty-first aspect, in addition to one or more of the preceding or following aspects, or in the alternative to some aspects, the probe shaft can be at least partially formed from a non-conductive material.

In a thirty-second aspect, in addition to one or more of the preceding or following aspects, or in the alternative to some aspects, the probe shaft can include a cylindrical body.

In a thirty-third aspect, in addition to one or more of the preceding or following aspects, or in the alternative to some aspects, the probe shaft can include a tubular body.

In a thirty-fourth aspect, in addition to one or more of the preceding or following aspects, or in the alternative to some aspects, the controller can be further configured to initiate an alarm or corrective action if a liquid level crosses a predetermined threshold value.

In a thirty-fifth aspect, in addition to one or more of the preceding or following aspects, or in the alternative to some aspects, the controller can be further configured to initiate sending a control signal to at least one of a pump or a valve if a liquid level crosses a predetermined threshold value.

In a thirty-sixth aspect, in addition to one or more of the preceding or following aspects, or in the alternative to some aspects, the controller can be further configured to log calculated liquid level data over time.

In a thirty-seventh aspect, in addition to one or more of the preceding or following aspects, or in the alternative to some aspects, can further include one or more secondary sensing elements, wherein the one or more secondary sensing elements can be disposed within or along the probe shaft.

In a thirty-eighth aspect, in addition to one or more of the preceding or following aspects, or in the alternative to some aspects, the one or more secondary sensing elements can include at least one selected from the group consisting of a temperature sensor, an optical sensor, an electrochemical sensor, a pH sensor, and a dissolved oxygen sensor.

In a thirty-ninth aspect, in addition to one or more of the preceding or following aspects, or in the alternative to some aspects, the probe shaft can be disposable and the probe shaft can be detachable from the controller.

In a fortieth aspect, in addition to one or more of the preceding or following aspects, or in the alternative to some aspects, can further include an electrical grounding lead, wherein the electrical grounding lead passes through or along a lengthwise axis of the probe shaft.

In a forty-first aspect, in addition to one or more of the preceding or following aspects, or in the alternative to some aspects, the plurality of conductive probe wires include a stainless-steel conductor.

In a forty-second aspect, in addition to one or more of the preceding or following aspects, or in the alternative to some aspects, the controller can be configured to periodically perform a self-calibration routine.

In a forty-third aspect, in addition to one or more of the preceding or following aspects, or in the alternative to some aspects, can further include a user interface, wherein the user interface displays real-time data and alerts regarding the liquid level and foam presence.

In a forty-fourth aspect, in addition to one or more of the preceding or following aspects, or in the alternative to some aspects, the controller can be further configured to estimate a volume of a liquid within the piece of bioprocess equipment based on the calculated liquid level and known dimensions of the piece of bioprocess equipment.

In a forty-fifth aspect, in addition to one or more of the preceding or following aspects, or in the alternative to some aspects, the multi-level liquid and foam sensor for bioprocess equipment can be configured for use with single use bioreactors.

In a forty-sixth aspect, in addition to one or more of the preceding or following aspects, or in the alternative to some aspects, the bioprocess equipment can be a bioreactor.

In a forty-seventh aspect, a method of monitoring a liquid level within a piece of bioprocess equipment can be included. The method can include sensing electrical properties of a plurality of conductive probe wires and estimating a liquid level within the piece of bioprocess equipment based on the sensed electrical properties.

In a forty-eighth aspect, in addition to one or more of the preceding or following aspects, or in the alternative to some aspects, the method can further include detecting a height of liquid in the vessel using the sensed electrical properties of at least two conductive probe wires.

In a forty-ninth aspect, in addition to one or more of the preceding or following aspects, or in the alternative to some aspects, the method can further include detecting the presence of foam above the liquid level using the sensed electrical properties.

In a fiftieth aspect, in addition to one or more of the preceding or following aspects, or in the alternative to some aspects, the method can further include detecting a height of foam above the liquid level using the sensed electrical properties of at least two conductive probe wires.

In a fifty-first aspect, in addition to one or more of the preceding or following aspects, or in the alternative to some aspects, the method can further include adjusting a sensitivity of foam detection based on the conductivity of the liquid within the piece of bioprocess equipment.

In a fifty-second aspect, in addition to one or more of the preceding or following aspects, or in the alternative to some aspects, the method can further include calculating a liquid level based on differing electrical properties between two conductive probe wires.

In a fifty-third aspect, in addition to one or more of the preceding or following aspects, or in the alternative to some aspects, the method can further include calculating a liquid level based on differing electrical properties between two adjacent conductive probe wires.

In a fifty-fourth aspect, in addition to one or more of the preceding or following aspects, or in the alternative to some aspects, the method can further include detecting the presence of foam above the liquid level based on differing electrical properties between two conductive probe wires.

In a fifty-fifth aspect, in addition to one or more of the preceding or following aspects, or in the alternative to some aspects, the method can further include detecting the height of foam above the liquid level based on differing electrical properties between two conductive probe wires.

In a fifty-sixth aspect, in addition to one or more of the preceding or following aspects, or in the alternative to some aspects, the method can further include detecting the type of foam based on differing electrical properties between two conductive probe wires.

In a fifty-seventh aspect, in addition to one or more of the preceding or following aspects, or in the alternative to some aspects, the method can further include sending data regarding the liquid level and foam presence to a remote processing unit.

In a fifty-eighth aspect, in addition to one or more of the preceding or following aspects, or in the alternative to some aspects, the method can further include initiating an alarm or corrective action if a liquid level crosses a predetermined threshold value.

In a fifty-ninth aspect, in addition to one or more of the preceding or following aspects, or in the alternative to some aspects, the method can further include initiating sending a control signal to at least one of a pump or a valve if a liquid level crosses a predetermined threshold value.

In a sixtieth aspect, in addition to one or more of the preceding or following aspects, or in the alternative to some aspects, the method can further include logging calculated liquid level data over time.

In a sixty-first aspect, in addition to one or more of the preceding or following aspects, or in the alternative to some aspects, can further include periodically performing a self-calibration routine.

In a sixty-second aspect, in addition to one or more of the preceding or following aspects, or in the alternative to some aspects, the method can further include estimating a volume of a liquid within the piece of bioprocess equipment based on the calculated liquid level and known dimensions of the piece of bioprocess equipment.

In a sixty-third aspect, in addition to one or more of the preceding or following aspects, or in the alternative to some aspects, the piece of bioprocess equipment can be a bioreactor.

In a sixty-fourth aspect, a piece of bioprocess equipment can be included having a vessel with non-conductive walls defining an internal volume and a multi-level liquid and foam sensor including a probe shaft configured to pass into the internal volume of the vessel. The sensor can include a plurality of conductive probe wires. The plurality of conductive probe wires can be arranged at different vertical levels along a height of the probe shaft. The sensor can include a controller that can be in electrical communication with the plurality of conductive probe wires. The controller can be configured to sense electrical properties of the plurality of conductive probe wires and estimate a liquid level and/or detect the presence of foam above the liquid level within the piece of bioprocess equipment based on the sensed electrical properties.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims and their legal equivalents.

### Brief Description of the Figures

Aspects may be more completely understood in connection with the following figures (FIGS.), in which:
FIG. 1 is a schematic view of a bioreactor system in accordance with various embodiments herein.
FIG. 2 is a schematic view of a bioreactor housing in accordance with various embodiments herein.
FIG. 3 is a schematic view of components of a bioreactor in accordance with various embodiments herein.
FIG. 4 is a schematic view of electrical contacts along a probe shaft of a sensor in accordance with various embodiments herein.
FIG. 5 is a schematic view of a sensor within a bioreactor in accordance with various embodiments herein.
FIG. 6 is a schematic view of a sensor within a bioreactor in accordance with various embodiments herein.
FIG. 7 is a table of exemplary electrical property values in accordance with various embodiments herein.
FIG. 8 is a schematic view of a portion of a probe shaft of a sensor in accordance with various embodiments herein.
FIG. 9 is a schematic view of a probe shaft of a sensor in accordance with various embodiments herein.
FIG. 10 is a schematic view of a liquid sensor for bioreactors in accordance with various embodiments herein.
FIG. 11 is a schematic view of a bioreactor system in accordance with various embodiments herein.
FIG. 12 is a schematic view of a bioprocess equipment system in accordance with various embodiments herein.

While embodiments are susceptible to various modifications and alternative forms, specifics thereof have been shown by way of example and drawings, and will be described in detail. It should be understood, however, that the scope herein is not limited to the particular aspects described. On the contrary, the intention is to cover modifications, equivalents, and alternatives falling within the spirit and scope herein.

### Detailed Description

Monitoring liquid (including, for example, buffers and cell culture media) levels within bioreactors and various other pieces of bioprocess equipment can aid in maintaining optimal conditions for bioprocesses, biological reactions, and product yield. Traditional methods for monitoring these levels have relied on various types of sensors, including optical, ultrasonic, and mechanical float sensors. However, the use of existing sensor systems with bioreactors has encountered various challenges. One significant challenge is the detection of foam. Foam can be generated during bioprocesses by proteins and lipids and can result in degradation of the product of interest trapped in the foams. Also, foam can clog gas vents and cause problems with pumps. Another challenge to address is the differentiation between foam and liquid levels.

Additionally, the conditions within bioprocess equipment, including associated temperatures and exposure to media, can limit how well certain types of sensors can work. Further challenges include that the bioprocess equipment needs to maintain aseptic conditions and that measuring equipment should not interact with or contaminate the liquid.

Embodiments herein relate to multi-level liquid and foam sensors designed to accurately monitor liquid and foam levels within various pieces of bioprocess equipment such as bioreactors (stirred tank, fixed bed, packed bed, fluidized bed, bubble column, airlift, bag bioreactors, photobioreactors, and the like), cartridges, media containers, bioprocess related tanks (storage, bulk, buffers, etc.), TFF (tangential flow filtration) concentration vessels, other liquid containing bioprocess equipment pieces, and the like. Such bioprocess equipment can specifically include disposable bioprocess equipment. Embodiments of multi-level liquid and foam sensors herein can specifically be configured for use with bioprocess equipment with polymeric vessel walls (e.g., without conductive walls) that is sterile or sterilizable. The sensor system can include a probe shaft, a plurality of conductive probe wires arranged in (such as molded into a wall), inside of (such as within an interior portion or a lumen), on, and/or otherwise along the probe shaft, and a controller in electrical communication with the conductive probe wires. The probe shaft can pass into a bioprocess vessel in order to measure one or more of the presence of liquid, liquid levels, the presence of foam, and foam levels. In some embodiments, a probe shaft herein can be integrated directly into the walls of a bioprocess vessel, but in other embodiments is separate from the walls. The controller can be configured to measure electrical properties such as one or more of conductance, resistance, capacitance, impedance, potential difference, or any relevant electrical signal of the conductive probe wires and estimate the liquid level and foam presence and/or level within the bioprocess equipment based on these sensed electrical properties.

The probe shaft can serve as the structural backbone of the sensor to which the conductive probe wires and/or electrical contacts are attached. The probe shaft can take on various forms, including cylindrical or tubular bodies, flat bodies, and the like, and sizes to accommodate different bioprocess equipment designs and sizes. The probe shaft can be configured to be either reusable or disposable, depending on the specific requirements of the bioprocess equipment or the vessel operation, with disposable versions being particularly suited for single-use bioreactors or other bioprocess vessels, such as disposable bioprocess vessels. Further, in some cases, certain portions of the sensor can be disposable (such as the probe shaft) while other portions (such as probe wires, electrical wires, cables, etc.) can be reusable. Further details regarding exemplary probe shafts are provided in greater detail below.

The conductive probe wires can be arranged along the probe shaft to have ends thereof exposed to the inside of the bioprocess equipment or vessel. In various embodiments, the conductive probe wires can be arranged at different vertical levels along the height of the probe shaft. In many cases, the wires can pass through the interior of the probe shaft (which could be filled or hollow) before passing to the outside thereof so as to include an exposed end portion that functions as an electrode as part of a measurement circuit and is in contact with the interior of the bioprocess equipment. In other embodiments, the wires can pass along a surface of the probe shaft with portions thereof being covered by an insulator and then also having an exposed end portion. Exposed end portions of wires herein can be fully exposed (such as bare metal), but in some embodiments can be covered with or otherwise coated by a layer of a biocompatible material (such as a biocompatible polymer or another material). The exposed end portions can be disposed in various configurations, including spiral, flat, curved, straight line, standing out from the surface or fitting within grooves (or a recess or some other carveout) on the exterior surface of the probe shaft so as to be substantially flush therewith. In some embodiments, exposed end portions of wires herein can be either partially or completely wrapped around the outside surface of the probe shaft. Further details of exemplary conductive probe wires are provided below.

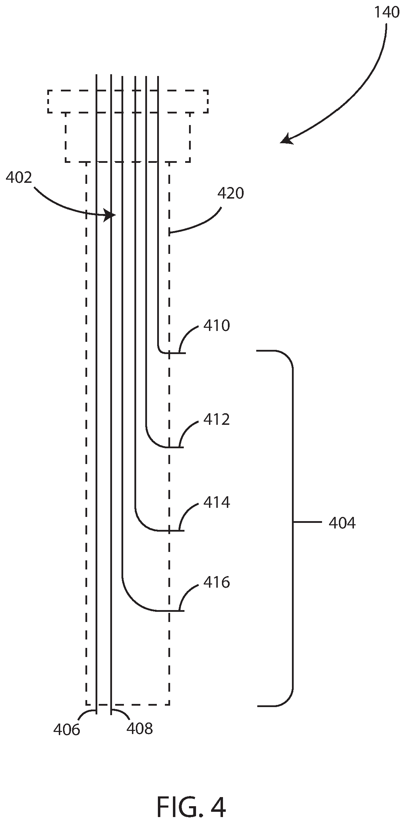

The controller can serve as the brain of the sensor system, configured to sense the electrical properties of the conductive probe wires and estimate the liquid level and foam presence and/or level within the bioprocess equipment. It can include various hardware such as a processor, a transmitter, a power supply circuit, and a communications circuit, such as to enable wired or wireless communications. The controller can execute operations to detect the height of liquid and foam by analyzing the sensed electrical properties of conductive probe wires, such as between at least two conductive probe wires and/or between a conductive probe wire and a common measurement electrode. In various embodiments, the controller can be configured to adjust the sensitivity of foam detection based on the conductivity of the particular liquid being used within the bioprocess equipment, which can be determined using conductive probe wires disposed adjacent to the bottom of the probe shaft (see, e.g., bottom contacts 406 and 408 of FIG. 4).

Additionally, in various embodiments, the controller can initiate and/or generate alarms, corrective actions, or control signals to pumps or valves if the liquid level crosses predetermined threshold values. It can be configured to determine the volume of liquid based on the height and the geometry of the system. It can also be configured to log calculated liquid level data over time and/or perform self-calibration routines periodically. A wireless transmitter can be incorporated to send data regarding the liquid level and foam presence to a remote processing unit, enhancing the flexibility and convenience of monitoring.

In some embodiments, a user interface can be provided to display real-time data and alerts regarding the liquid level and foam presence, allowing operators to make informed decisions promptly.

The sensor system can include a connection fitting at the top of the probe shaft for engagement with ports, such as standardized ports, in the housing of bioprocess equipment, such as a PDG 13.5 port or another type of standardized port. In various embodiments, sensor systems herein are configured for use with pieces of bioprocess equipment that have a housing made from a non-conductive material, such as polymer or a composite material. The system may also include one or more secondary sensing elements, such as temperature, optical, electrochemical, pH, and dissolved oxygen sensors, integrated within or along the probe shaft for comprehensive monitoring of the bioprocess equipment's conditions.

The term "bioreactor" as used herein means a physical device, which biologically active environment thereof is suitable for cultivation of micro-organisms performing a desired process as disposed in or suspended in liquid media, typically agitated such as by an impeller or through other means.

The terms "media", "growth media", and "nutrient" as used herein are used interchangeable and refers to a sterile complex mixture containing mostly water, carbon sources, various gases such as oxygen and additives such as vitamins, hormones, growth factors, animal serum, antibiotics, antioxidants, antifoams, cell stabilizers and other components for cultivation of "micro-organisms". Some media are serum based, some are serum free, animal free, and protein free or chemically defined media. During cultivation the combined media and micro-organism and various debris is named "broth".

The term "buffer" or "buffers" as used herein refers to an aqueous solution that is formulated to maintain a specific pH or ionic strength during a bioprocess, and is typically comprised of a weak acid and its conjugate base, or a weak base and its conjugate acid, in solution. Buffers may also contain additional salts, stabilizers, or other excipients to ensure compatibility with bioprocess operations or the stability of biological products.

The term "ports" as used herein refers to holes anywhere in a wall allowing so attachment of suitable fittings or relevant sensors or general connections selected from the group of; PG 13.5 thread ports, locking ports, press-in ports, or ports involving Luer-Loc fitting, connecting fittings, sterile fittings, hoses, tubes, hose barbs, etc.

The term "PCS" or "Process-Control-System" as used herein refer to a Programmable Logic Control (PLC), a Personal Computer (PC) an Embedded Computer (EC) both integrating a Central-Processing-Unit (CPU) electronic device with calculating power. The PLC has various Input & Output (I/O) with input from various sensors and output for various actuators for process control purposes. The PCS integrate software, algorithms, process recipe in build-in memory for managing, analysis of the cultivation or fermentation process for on-going process alignment according to the process recipe. The PCS communicate with a variety of sensors and actuators in order to alter the process parameters.

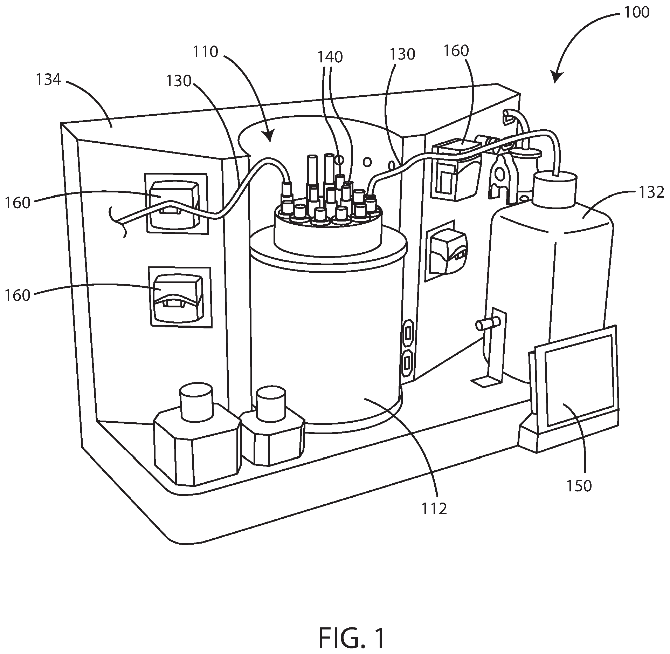

FIG. 1 illustrates a schematic view of a bioreactor (as a specific example of a piece of bioprocess equipment), such as in the form of a cell culture system 100 equipped with the multi-level liquid and foam sensor system of the present invention. The system 100 includes a cell culture vessel or device, such as for example a bioreactor 110 which includes a housing 112 made of an electrically insulating material, such as a polymer in the case of a disposable bioreactor. However, it will be appreciated that the housing 112 could also be made of metal, glass, composite, or combinations of materials, particularly in the case of a non-disposable bioreactor. One or more sensors 140 may also be associated with the bioreactor 110, such as the liquid and foam sensors described herein as well as pH, dissolved oxygen, temperature, or cell density/biomass sensors, or sensors for other desirable parameters, can also be provided. The system 100 can also include tubing lines 130, which can be connected to a reservoir, such as a bottle 132, for culture media supply and/or circulation. Fluid flow through the system can be under the control of pumps 160, which could be peristaltic pumps or another type of pump.

The system 100 can also include a controller 134 for providing process control functionalities (such as by controlling pumps 160, valves, heating elements, and the like) and standard parameter monitoring. The controller 134 can be or be part of a Process-Control-System (PCS) as described elsewhere herein. The controller 134 can be in signal communication with the sensor(s) 140 (or sensor system(s)) to allow for monitoring one or more process parameters. The controller 134 may be connected to a display 150 which can include a user interface thereon for displaying key parameters and other aspects for control of the system 100.

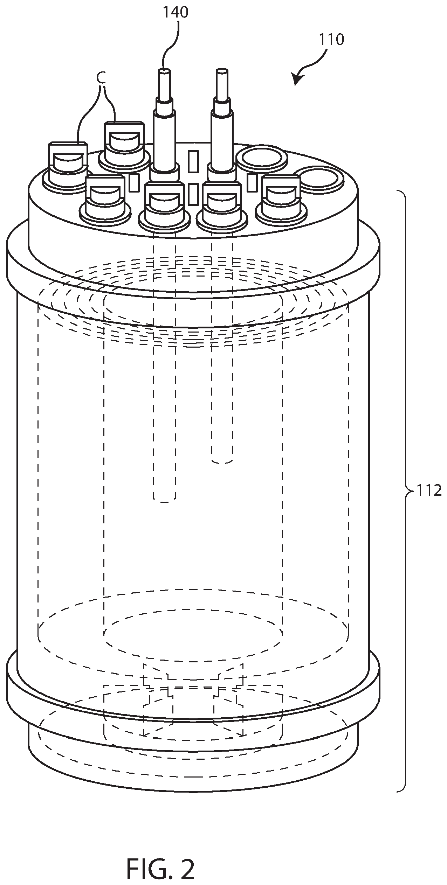

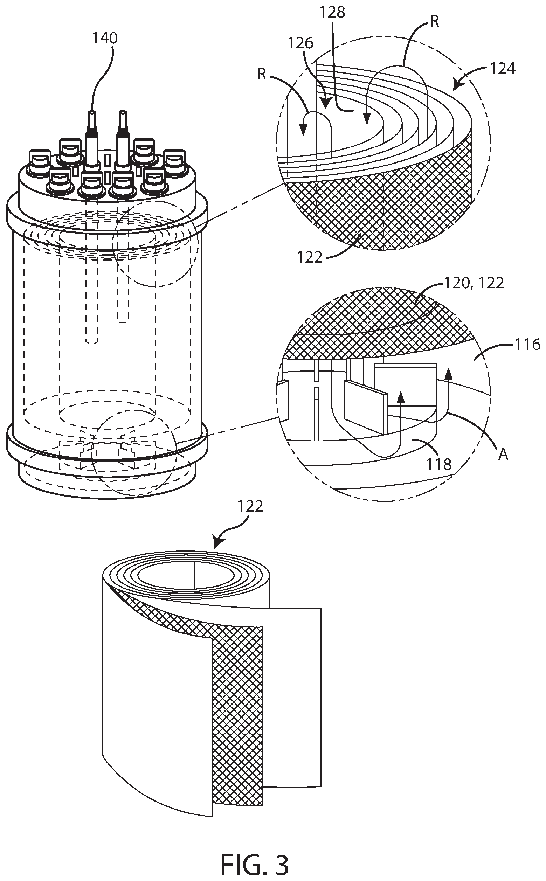

FIG. 2 is a schematic view of a bioreactor housing 112 in accordance with various embodiments herein. FIG. 3 is a schematic view of components of a bioreactor in accordance with various embodiments herein. With reference to FIGS. 2 and 3, the bioreactor 110 includes a housing 112 in which one or more compartments or chambers exist for transmitting a flow of fluid, gas, or both, as well as a sensor 140 as described herein. As shown, a first chamber 116 at or near a base of the bioreactor housing 112 is provided. In some embodiments, the first chamber 116 may include an agitator for causing fluid flow. The agitator may, for example, be in the form of a magnetically driven impeller 118, in which case it may further contribute to the issue of electromagnetic noise. However, the agitator 118 could alternatively or additionally be in the form of an impeller with a mechanical coupling to the base, an external pump forming part of a fluid circulation system, or any other device for causing fluid circulation within the bioreactor.

In the type of system illustrated, liquid exiting the chamber 120 passes to a headspace formed by a chamber 124 on one (upper) side of the bed 122, where the liquid is exposed to a gas (such as oxygen). In some embodiments, the bed 122 and include a solid or porous carrier or scaffold to attach adherent cells. In some embodiments, liquid may then flow radially inwardly to a central chamber 126 to return to the lower entrance of bed 122. The liquid can be a nutrient-rich fluid that flows through the bed 122 to provide for the cells. In some embodiments, this central chamber 126 can be columnar in nature and may be formed by an imperforate conduit or tube 128 or rather formed by the central opening of the structured spiral bed 122. The chamber 126 returns the liquid to the first chamber 116 (return arrow R) for recirculation through the bioreactor 110, such that a continuous loop results ("bottom to top" in this version).

Sensor systems herein can include a probe shaft, a plurality of conductive probe wires arranged inside and/or along the probe shaft, and can be used with bioreactors such as the type shown in FIGS. 1-3 or of other types. Referring now to FIG. 4, a schematic view of probe wires and/or electrical contacts along a probe shaft of a sensor is shown in accordance with various embodiments herein. In specific, FIG. 4 provides a depiction of the multi-level liquid and foam sensor for bioreactors 140 with a schematic view of the probe shaft 420 and associated conductive probe wires and exposed portions or electrical contacts thereof. This figure schematically illustrates possible arrangement and functionality of the conductive probe wires 402 and their specific placements for sensing within a bioreactor environment.

The sensor system and/or components thereof such as the probe shaft and probe wires can be configured to be sterilizable by gamma irradiation, autoclaving, ethylene oxide sterilization, or the like. In various embodiments, the sensor system and/or components thereof can be sterilized at the same time as the bioreactor or bioprocess vessel which the sensor system is used with.

The probe shaft 420 is illustrated in FIG. 4 as a structure extending vertically down into the main chamber of the bioreactor. It serves as the element to which the conductive probe wires 402 are attached. Materials of the probe shaft 420 can be selected to ensure compatibility and functionality within the bioreactor environment, accommodating a variety of bioprocessing applications. In some embodiments, the probe shaft 420 can be constructed from durable, non-conductive materials such as various polymers or composite materials that can withstand conditions commonly found within bioreactors. In some embodiments, materials can be selected for their chemical inertness, thermal stability, and mechanical strength, ensuring that the probe shaft maintains its structural integrity and electrical isolation properties over periods of use. In some embodiments, the shaft can also be made from conductive materials and the probe wires 402 can be electrically insulated from the same.

Components of the sensor such as the probe shaft 420 and other components can be manufactured using various techniques including, but not limited to, 3D printing (or deposition printing), molding, injection molding, blow molding, vacuum-forming, and the like. Materials used to form the probe shaft 420 and/or various other components of the sensor can include, but are not limited to, materials such as; polycarbonates, polyesters, nylons, polyamide, PTFE resins and other fluoropolymers, acrylic and methacrylic resins and copolymers, polysulphones, polyethersulphones, polyarylsulphones, polystyrenes, polyetherimides, polyethylene terephthalates, polyvinyl chlorides, chlorinated polyvinyl chlorides, ABS and its alloys and blends, polyolefins, such as polyethylenes including linear low density polyethylene, low density polyethylene, high density polyethylene, and ultrahigh molecular weight polyethylene and copolymers thereof, polypropylene and copolymers thereof and metallocene generated polyolefins. In some embodiments, semi-rigid materials such as silicone, rubber, or various elastic materials can be used.

The probe shaft 420 can be attached to other components, such as to a connection fitting at the top of the bioreactor, through a variety of means including threaded connections, an interlock mechanism, snap-fit or compression fit mechanisms, or even adhesive bonding. This allows for easy installation and removal from the bioreactor, facilitating maintenance and replacement processes.

In some embodiments, sealing of parts between assembling surfaces can include the use of elastomeric element(s) such as an O-ring or flat washers or diaphragms that could be nitrile rubber, silicone rubber, Viton rubber, latex rubber, EPDM or other elastic material. In some embodiments, sealing of parts between assembling surfaces can be performed with adhesive materials such as UV curing adhesives, epoxies, or the like, or combinations thereof.

In terms of functionality, the probe shaft 420 can be designed to house and protect the conductive probe wires while ensuring their precise positioning within the bioreactor. The probe shaft 420 can include internal channels or external grooves along its length, which can serve to route the conductive probe wires from the top of the shaft to their designated positions along the shaft's exterior. These channels or grooves can be configured to prevent the wires from shifting, ensuring consistent sensor performance.

The probe shaft 420 can be manufactured in various shapes and sizes to accommodate different bioreactor designs and capacities. Common shapes include cylindrical and tubular forms, with diameters and lengths tailored to specific bioreactor dimensions. The flexibility in design allows the sensor system to be utilized in a wide range of bioreactor sizes, from small laboratory-scale units to larger scale systems.

Positioning and orientation of the probe shaft 420 within the bioreactor can contribute to accurate sensing. The shaft is typically positioned vertically, extending from a top portion of the bioreactor towards its bottom, to allow the conductive probe wires to interact effectively with the bioreactor's contents across different levels. This vertical orientation facilitates estimating liquid (or media) height within the bioreactor. However, other positions and orientations are also contemplated herein.

In some embodiments, the versatility of the probe shaft 420 is further enhanced by the possibility of incorporating secondary sensing elements along its length. These elements can include temperature, pH, and dissolved oxygen sensors, among others, which are integrated within or along the probe shaft. This integration allows for comprehensive monitoring of the bioreactor's conditions, providing valuable data for monitoring and/or controlling bioprocessing operations. It also reduces the number of probes that would otherwise be required which provides benefits in terms of maintaining sterility and enhancing simplicity. In various embodiments, one or more secondary sensing elements can be attached at or near the bottom of the probe shaft 420. However, in various embodiments, one or more secondary sensing elements can also be attached at or near other points of the probe shaft 420.

FIG. 4 shows a plurality of conductive probe wires 402. These wires are strategically positioned at various vertical levels along the probe shaft 420. Each wire is isolated from the others. Each wire is shown with an exposed end portion, designed to interact directly with the bioreactor's internal environment. This direct interaction facilitates the sensing of electrical properties such as conductance or conductivity, resistance or resistivity, capacitance, impedance, and the like which can be used for estimating liquid and/or foam levels and detecting foam presence.

The conductive probe wires 402 can be made from a variety of materials known for their conductive properties, including but not limited to, stainless steel, copper, aluminum, titanium, gold, platinum, and the like. These materials are selected based on their durability, resistance to corrosion, and compatibility with the bioreactor environment, which often includes exposure to various biochemical substances and conditions. In some embodiments, the conductive probe wires can be one material inside the probe wire and can be coated with stainless steel or another inert metal such as titanium, gold, platinum, or others outside the probe wire. In some embodiments, the conductive probe wires can be one material inside the probe shaft and can be or can be coated with another metal such as stainless steel or another inert metal such as titanium or others outside the probe shaft (e.g., the portion exposed to the inside of the bioreactor with liquid or media can be an inert material).

Attachment of the conductive probe wires 402 to the probe shaft 420 can be achieved through various techniques, including using adhesives, molding in place, the use of various fasteners, and the like, depending on the material of the probe wires and the probe shaft.

The conductive probe wires 402 and/or contacts can be positioned in varying configurations around or along the probe shaft 420 to enhance measurement accuracy and reliability within diverse bioreactor contexts. For example, the wires and/or contacts can be arranged in a spiral, straight, or staggered patterns, each offering different benefits in terms of sensor coverage and the ability to detect changes in the bioreactor's internal environment. The positioning strategy can be selected based on the specific monitoring requirements of the bioreactor, taking into consideration factors such as the size of the bioreactor, the type of bioprocess being conducted, and the expected range of liquid levels. One approach involves arranging the conductive probe wires in a helical pattern that runs longitudinally along the shaft. This configuration can increase the coverage surface area of the probe wire, allowing for more comprehensive interaction with the medium within the bioreactor. The helical pattern not only provides multiple touchpoints for sensing along the vertical axis but also helps to minimize discrepancies arising from uneven liquid distribution or foam stratification. In another approach, the probe wires and/or contact can be staggered from one side to the other along the vertical dimension of the probe shaft 420 at different vertical positions such that adjacent exposed ends of probe wires or contacts are disposed on opposite sides of the probe shaft 420 providing an increase in distance (greater than the vertical offset distance) between adjacent exposed ends or contacts.

The sizes of the conductive probe wires 402 can vary, with diameters typically ranging from a few micrometers to millimeters, depending on various factors including the required sensitivity and the physical constraints of the bioreactor design. The length of the exposed end portions of the wires is also variable, typically ranging from 0.5 to 3 centimeters, to ensure adequate exposure to the bioreactor's contents. However, other lengths of exposed end portions are also contemplated herein.

FIG. 4 illustrates an exemplary vertical sensing height range 404. The sensing height range 404 represents the total vertical range over which the conductive probe wires 402 can be used to sense the presence of liquid and/or foam. The height range 404 can vary based on what is relevant for the particular bioreactor the sensor is used with. However, in various embodiments, the height range 404 can be from 0.1 millimeter to 50 centimeters or more, or in some embodiments from 2 to 10 millimeters.

In some embodiments, the sensor can include first and second bottom contacts 406, 408 (which can be the ends of probe wires) positioned at the lower end of the probe shaft 420. In some cases, their placement at a point that is planned to be within liquid makes them useful for establishing a baseline electrical property of the liquid, which aids in the accurate sensing of liquid levels and foam presence.

In this example, first contact 410, second contact 412, third contact 414, and fourth contact 416 are also depicted. These contacts are distributed at different vertical levels along the probe shaft 420, each serving as a point for sensing the electrical properties indicative of liquid and foam. The placement of probe wires at different specific heights can ensure that electrical property measurements cover a range of relevant operational scenarios, providing comprehensive monitoring capabilities across diverse bioprocess conditions. The side contacts are shown with their exposed end portions extending outward, ensuring effective interaction with the bioreactor's contents. Their staggered arrangement can allow for precise detection of changes in liquid level and the differentiation between liquid and foam based on electrical properties.

The number of conductive probe wires and/or contacts can vary. In some embodiments, the sensor can include at least 2, 3, 4, 5, 6, 7, 8, 9, 10, 12, 15 or more probe wires and/or corresponding exposed ends or contacts, or a number falling with within a range between any of the foregoing. The distance between adjacent ends or contacts (or between each sensing level) can be from 0.1 millimeters to 500 millimeters, or in some embodiments from 2 to 10 millimeters.

As will be described in greater detail below, the conductive probe wires 402 can be connected to a controller (not shown in FIG. 4), which processes the electrical signals to estimate the liquid level, foam presence, the foam level, and potentially the foam type. As such, data from the probe wires can be analyzed to provide real-time monitoring and control of the bioreactor's internal conditions.

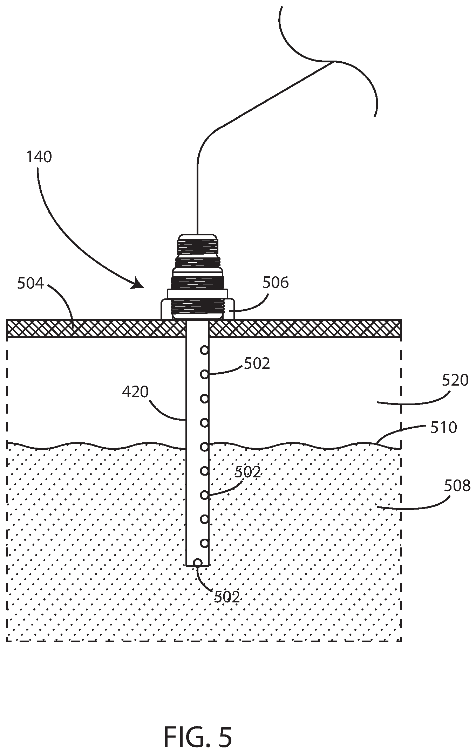

Referring now to FIG. 5, is a schematic view of a sensor 140 within a bioreactor in accordance with various embodiments herein. This figure provides a partial cross-sectional view of the bioreactor, highlighting the integration of the sensor system, specifically the probe shaft 420, and its interaction with the bioreactor's contents, including liquid 508 and air 520. The figure illustrates the sensor system's functionality in detecting liquid height 510 and differentiating between liquid and air within the bioreactor.

FIG. 5 shows a portion of the housing 504 of the bioreactor. The housing material for disposable bioreactors is typically made from a non-conductive material such as a polymer, glass, or composite. However, the bioreactor housing 504 could also be made of other materials such as a metal or combination of materials in some embodiments. The housing ensures a controlled environment for bioprocessing activities.

At the top of the housing 504, a standardized port 506 is shown, which serves as the entry point for the probe shaft 420 of the sensor system. This port 506 is designed to accommodate the connection fitting of the probe shaft, ensuring a secure and stable installation. The standardized port 506 facilitates easy integration of the sensor system into various bioreactor models and designs. In some embodiments, the standardized port 506 can include an interlock mechanism or other structure to ensure secure engagement of the sensor 140 with the standardized port 506.

Extending vertically from the standardized port 506 into the bioreactor, the probe shaft 420 is a central component of the sensor system. It is depicted as being immersed in the bioreactor's contents, with its lower end reaching into the liquid 508. In some embodiments, the probe shaft 420 extends to the bottom of the bioreactor for empty detect purposes and/or the end of the probe wire can reside in a sump cavity somewhere in the bioreactor. The probe shaft can be made from a non-conductive material and houses the conductive probe wires (not shown in FIG. 5) that are used for sensing the electrical properties related to liquid height and foam presence. A plurality of contacts or exposed end portions 502 of probe wires are shown arranged to be spaced out along the vertical dimension of the probe shaft 420.

The bioreactor's contents are partially illustrated, with the liquid 508 shown occupying the lower portion of the housing 504. This liquid represents the medium or culture fluid within which bioprocessing activities occur. The vertical distance from the bottom of the bioreactor to the surface of the liquid 508 is depicted, representing the liquid height 510. This measurement is significant as it provides the basis for estimating the volume of liquid within the bioreactor and monitoring changes in liquid level during bioprocessing operations. Above the liquid 508, the space filled with air (or potentially partially filled foam) is shown, indicating the interface between the liquid and the air within the bioreactor.

While FIG. 5 shows the sensor system mounted on top of the bioreactor vessel, with the probe shaft extending down into the same, it will be appreciated that the probe shaft of sensor systems herein can be adapted for connection to the bioreactor vessel through the lid, a custom port, a sidewall, a base, etc. Further, in some embodiments, the probe shaft can even reside in a side vessel or tube with a fluid level that mimics the level with the bioreactor vessel.

In various embodiments, the sensor system herein is configured to provide real-time monitoring of the liquid height 510. Thus, by sensing the electrical properties of the environment within the bioreactor, the sensor system can provide important data for the efficient management and control of bioprocessing activities.

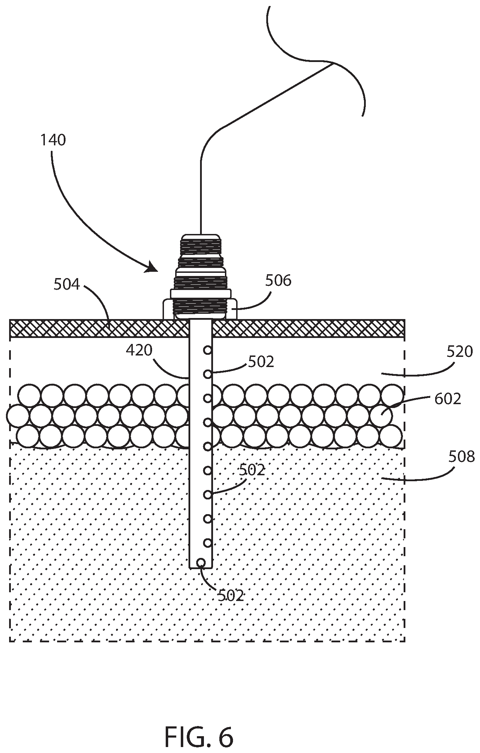

As described elsewhere herein, sensor systems of embodiments herein can also be used to detect the presence of foam and/or the height of foam. FIG. 6 is a schematic view of a sensor within a bioreactor in accordance with various embodiments herein. This figure provides a partial cross-sectional view of a bioreactor equipped with the multi-level liquid and foam sensor system 140 focusing on the sensor's interaction with the bioreactor environment, which includes liquid 508, air 520, and foam 602. This figure is designed to illustrate the sensor system's capability to accurately monitor the liquid level, as well as detect and differentiate between the presence of foam and air above the liquid within the bioreactor.

Similar to that shown in FIG. 5, FIG. 6 shows the housing 504 of the bioreactor, standardized port 506, probe shaft 420, liquid 508, the air space 520, and the plurality of contacts or exposed end portions 502 of probe wires spaced out along the vertical dimension of the probe shaft 420. However, in this embodiment, a foam layer 602 is shown positioned between the liquid 508 and air 520. The foam layer's 602 electrical properties differ from those of liquid and air, enabling the sensor system to distinguish between these phases and accurately assess the presence of foam, foam height, and in some cases properties of the foam such as its density.

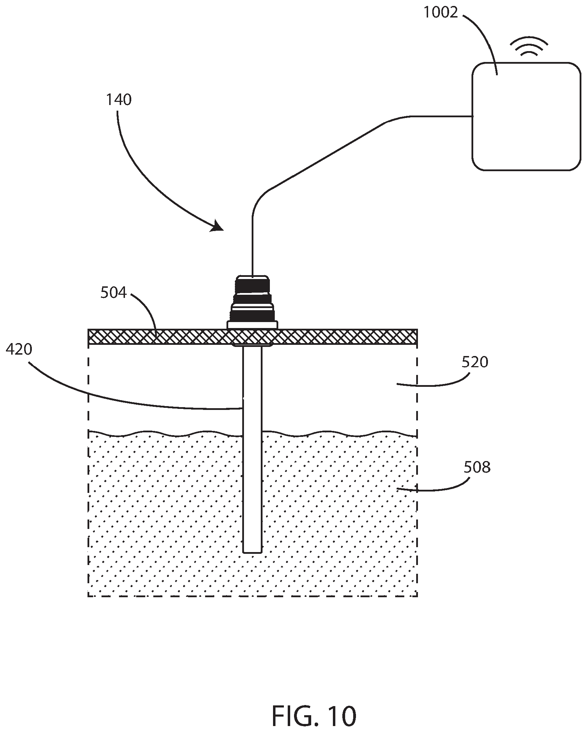

In an operational workflow of the multi-level liquid and foam sensor system, conductive probe wires and specifically exposed ends thereof or contacts are used to sense changes in electrical properties caused by variations in liquid level and the presence of foam. This data is processed by the system's controller (shown in FIG. 10) to provide real-time monitoring and control inputs for bioprocessing operations, such as adjusting agitation speed, antifoam agent dosing, operating pumps and/or valves, or other actions to monitor and/or manage liquid levels and/or foam presence and formation. In some embodiments, the data processed by the system's controller can be used to detect/monitor complete or partial filling operations and/or complete or partial emptying operations.

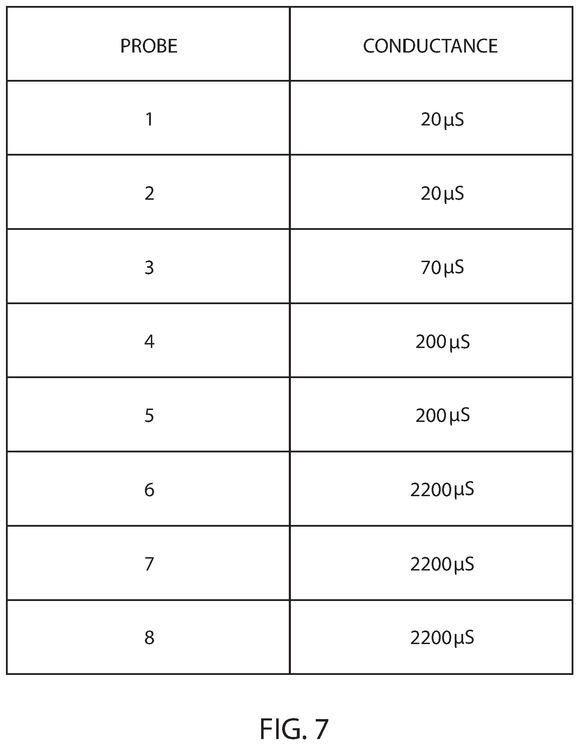

Referring now to FIG. 7, a table of exemplary electrical property values in accordance with various embodiments herein. The representation of electrical property values in FIG. 7 focuses on conductance measurements related to a multi-level liquid and foam sensor system for bioreactors. This table illustrates the conductance values associated with various probe or probe wire positions or levels along the probe shaft, offering insights into the sensor's performance in different conditions. It will be appreciated that the conductance values shown in FIG. 7 are only provided to aid in explanation of how the system works and are not necessarily reflective of a particular set of actual measurements made.

Electrical properties can be measured in various ways. In one approach, each probe wire at different heights acts as a contact point for evaluating electrical properties, such as conductance (or in some cases conductivity). Conductance values can be obtained by applying an electrical potential across a selected pair of probe wires (or one probe wire and a common measurement electrode), measuring the resulting current flow, and then using both the known electrical potential and measured current in an equation to calculate conductance (or another electrical property). For example, the equation G = I/V, where G is conductance in Siemens, I is the current in amps, and V is the voltage can be used for calculations. In various embodiments, the system can account and correct for the potential differences in distance between different probes (or probe wires) being used for measurements. In some embodiments, measurements of conductivity can be made. The measured electrical property data can provide direct insights into the material (liquid, foam, air) through which the current passes allowing for the detection of liquid, foam, and air at the heights of the selected probe wires. By analyzing varying electrical property values (such as conductance values) at different probe heights, the system can identify the interfaces between liquid, foam, and air.

In this example, the probe wires are numbered sequentially from 1 to 8, indicating their specific positions along the sensor's vertical axis. The second column of the table lists the conductance values measured in microsiemens (µS) for each respective probe wire. These values represent the electrical conductance detected by the sensor at each level, providing data used by the system's controller to estimate liquid levels and detect foam presence. In this example, probe wires 1 and 2 both show conductance values of 20 µS. Such relatively low conductance values can be indicative of probe wires 1 and 2 being in contact with air. As such, these values can indicate that liquid and foam levels are lower than the levels of probes 1 and 2.

Probe wire 3 shows a conductance value of 70 µS, which is somewhat higher than that of probe wires 1 and 2. This can be indicative of an increase in moisture at the level of probe wire 3 and/or partial immersion in foam. In contrast to probe wire 3, Probe wires 4 and 5 display conductance values of 200 µS, which can be indicative of full immersion within the foam on top of the liquid phase inside the bioreactor. As such, the foam height can be determined to be above probe wire 4 and up to the height of probe wire 3.

Probe wires 6, 7, and 8 each show much higher conductance values of 2200 µS, indicating that such probes are immersed within the liquid. As such, the liquid height can be determined to be between the height of probe wire 6 and probe wire 5.

As such, by comparing electrical property values across different probes (placed at different heights) the system can estimate liquid height, estimate foam height, differentiate between liquid and foam, and therefore allow operational parameters to be adjusted accordingly. For emphasis, the conductance values shown in FIG. 7 are only provided to aid in explanation of how the system works and actual measurements made in real life systems may vary.

In various embodiments, after proper calibration, as the level of liquid increases, the signal from the different wires will change. This makes it possible to determine the liquid level between two wires and achieve continuous estimation of the level using discrete points of measurement (e.g., estimation of the liquid surface height by interpolating between discrete probe positions based on measured electrical properties).

In some embodiments, electrical property values can be used to not only determine the heights of liquid and/or foam within the bioreactor, but also determine properties of the liquid and/or foam. For example, in some embodiments, the system can detect the type of foam present based on the electrical properties. For example, a dense foam with smaller bubble sizes can have electrical properties that are closer to those of a liquid than a less dense foam might have.

In some embodiments, the system can execute a self-calibration function to ensure the accuracy of measurements obtained from the sensors. In some embodiments, calibration can include performing specific routines or operations to align the sensed electrical properties with known reference standards across the operational range of the sensor. In practice, this can include calibrating for conductance, resistance, capacitance, and impedance values, which could vary not just due to the liquid or foam being sensed but also because of factors such as temperature fluctuations and chemical composition of the bioreactor contents.

During an exemplary calibration operation, a current can be applied to different probe wires at different levels on the probe shaft, and the resulting measured electrical property values can be recorded. These measured values can be compared against relevant established reference values, and then adjustments to the controller's computational algorithms can be made allowing the sensor system to account for any discrepancies. Through this process, any offsets or drifts in sensor readings due to environmental variances or component aging can be corrected for. For enhanced reliability, the sensor system's controller can be configured to execute self-calibration procedures periodically or whenever significant deviations are detected via the feedback from the sensors. In various embodiments, data herein can be collected and reused for future process optimization and future cell cultures.

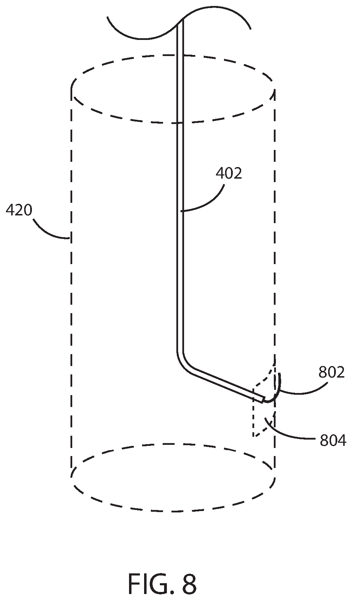

Referring now to FIG. 8, a schematic view of a portion of a probe shaft of a sensor is shown in accordance with various embodiments herein. In specific, FIG. 8 offers a detailed close-up view of the probe shaft 420, emphasizing the arrangement and specific features of the plurality of conductive probe wires 402, including their exposed end portions 802 and the outer surface 804 of the probe shaft. This figure illustrates possible structural details of the sensor components, highlighting their roles in the accurate detection of liquid levels and foam presence within a bioreactor.

The probe shaft 420 is depicted as the central structural element around which the sensor system is built. It is shown in a partial, zoomed-in view to focus on the details of its construction and the integration of the conductive probe wires. The outer surface 804 of the probe shaft is illustrated to provide context for the positioning of the conductive probe wires.

The plurality of conductive probe wires 402 (only a single probe wire is shown in FIG. 8 for ease of illustration) are strategically attached to the probe shaft 420, arranged to span various vertical levels along its length. The exposed end portion 802 of the conductive probe wire 402 is shown illustrating its configuration and placement relative to the probe shaft's outer surface 804. The exposed ends allow for the sensor's functionality, directly interacting with the bioreactor's internal environment to sense electrical properties indicative of liquid and/or foam.

The outer surface 804 of the probe shaft serves as the foundation for the attachment of the conductive probe wires and may feature grooves, channels, or other modifications to facilitate the precise placement and securement of the wires. In this example, the exposed end portion 802 at least partially wraps around the circumference of the outer surface 804 of the probe shaft. In some embodiments, the wrapped portion can be completely horizontal (such that the wrapped portion is perpendicular to the vertical dimension of the probe shaft or it can be angled with respect to horizontal. In some embodiments, the exposed end portion 802 can wrap around covering at least about 10, 20, 30, 45, 60, 75, or 90 degrees or more of the circumference of the probe shaft, or an amount falling within a range between any of the foregoing. The exposed end portion 802 can be proud from the outer surface 804 or fit within a groove or channel and can be substantially flush with the outer surface 804.

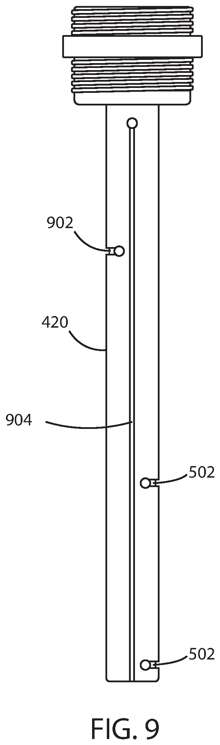

Referring now to FIG. 9, a schematic view of a probe shaft of a sensor in accordance with various embodiments herein. In specific, FIG. 9 provides a focused illustration of the probe shaft 420, detailing how features such as an opposite side contact and a common electrode can be used with the multi-level liquid and foam sensor system for bioreactors. As before, the probe shaft 420 is depicted as the main structural component of the sensor system. FIG. 9 also shows a plurality of contacts or exposed end portions 502 of probe wires.

FIG. 9 also shows an optional opposite side contact 902. This element is shown positioned on one side of the probe shaft 420, opposite a plurality of contacts or exposed end portions 502 of probe wires. The opposite side contact 902 can be designed to interact with the bioreactor's internal environment, but is positioned differently than contacts or exposed end portions 502, allowing for measurements to be taken from multiple sides of the probe shaft 420.

FIG. 9 also shows an optional common electrode 904, which is illustrated running vertically along the probe shaft 420. In some embodiments, the common electrode 904 can be used to pair with any of the exposed end portions or contacts when measurements of electrical properties are made. In other embodiments, the common electrode 904 may serve other purposes such as providing an electrical ground, particularly in embodiments wherein the housing of the bioreactor is made from an electrically insulating material such as a polymer or certain types of composites.

FIG. 10 illustrates a schematic view of the multi-level liquid and foam sensor system 140 integrated into a bioreactor environment. This figure highlights components and interactions within the system, specifically focusing on the arrangement of the probe shaft 420, the surrounding liquid 508, air 520, and a controller 1002. The bioreactor is depicted in a partial cross-sectional view, illustrating the internal environment where various biological processes occur. The lower portion of the bioreactor is occupied by the liquid 508, representing the culture medium or substrate used in bioprocessing applications. Above the liquid is the air space 520, illustrating the interface between the liquid and the gaseous environment within the reactor.

The sensor system 140 is shown penetrating the top surface of the housing 504 of the bioreactor. The probe shaft 420 extends vertically into the bioreactor, reaching from the air space down into the liquid 508. This configuration allows the conductive probe wires (not visible in FIG. 10) housed within or attached to the probe shaft to accurately sense electrical properties at different levels, facilitating the detection and monitoring of both liquid and/or foam levels and foam presence.

The probe shaft 420 is securely positioned within the bioreactor through a sealed connection at the top. This ensures the stability of the sensor system and prevents leakage or contamination of the bioreactor contents. The material of the probe shaft is typically non-conductive, providing electrical isolation for accurate sensor readings.

The controller 1002 is illustrated as being in electrical communication with the sensor system 140, receiving data transmitted from the conductive probe wires. The controller can be configured for processing and analyzing the sensed data, enabling real-time monitoring and control of the bioreactor's internal conditions. In some embodiments, data from the controller can be transmitted wirelessly to a remote processing unit, offering flexibility in monitoring and controlling bioprocess operations. In some embodiments, the controller 1002 can be the same as or part of the controller 134 referred to in FIG. 1, but in some embodiments, the controller 1002 of the sensor herein can be completely separate from the controller 134 referred to in FIG. 1 or only be in signal communication with the controller 134.

In various implementations, the controller 1002 can be configured to determine the respective heights of both liquid and foam within the bioreactor via the use of a plurality of conductive probe wires. This configuration enables the sensor system to effectively measure electrical properties, such as resistive, capacitive, or conductive characteristics, at different vertical locations along the probe shaft. By comparing the electrical properties sensed between different pairs of probe wires, the system can infer the liquid level. This comparison takes advantage of the different conductance values that distinguish between air, foam, and liquid environments, allowing the system to dynamically adjust its algorithms to various bioreactor contents.

The presence of foam within the bioreactor can represent a significant challenge to traditional monitoring systems, which might misinterpret foam as additional liquid due to their similar conductive properties. With the advanced multi-level sensor system described herein, the controller 1002 is configured not only to detect the presence of foam but also to determine its thickness above the liquid. By observing variations in sensed electrical properties between select pairs of conductive wires, the system can differentiate between foam layers and liquid, enabling more reliable and consistent process control. Data regarding liquid and foam levels can allow the controller 1002 and/or other components of the bioreactor to control bioreactor processes more effectively, such as by preventing excess foam and ensuring that optimal liquid levels are maintained.

It will be appreciated that in some cases the level of liquid and/or foam can be different in one part of the bioreactor (or other bioprocess equipment) than in another. For example, a liquid level above a bed may be different than a liquid level above a core or outer vessel portion of the bioreactor. In such cases, more than one sensor herein can be utilized and/or a sensor with more than one probe shaft can be utilized to be able to accurately detect fluid and/or foam levels in the various parts of the bioreactor.

Further, in certain embodiments, the foam detection mechanism includes a sensitivity adjustment feature that tunes sensor performance based on the conductivity of the liquid within the bioreactor. This adjustment ensures robustness in environments with diverse ionic compositions, adapting to changes that could affect sensing accuracy. By utilizing electrical property variations detected between adjacent probe wires, the sensor can also categorize the type of foam encountered, thus allowing for appropriate responses to mitigate foam-related issues.

To support continuous bioprocess monitoring and optimization, the sensor system's controller 1002 can function as a comprehensive data logger, recording liquid levels over time. This temporal data collection enables a historical analysis of bioreactor conditions, assisting in process validation and optimization as well as troubleshooting. In some embodiments, if liquid levels cross predetermined threshold values, indicating potential system failure or process deviations, the controller 1002 can be configured to initiate alarms or automatic corrective actions, such as signaling a pump or valve adjustment.

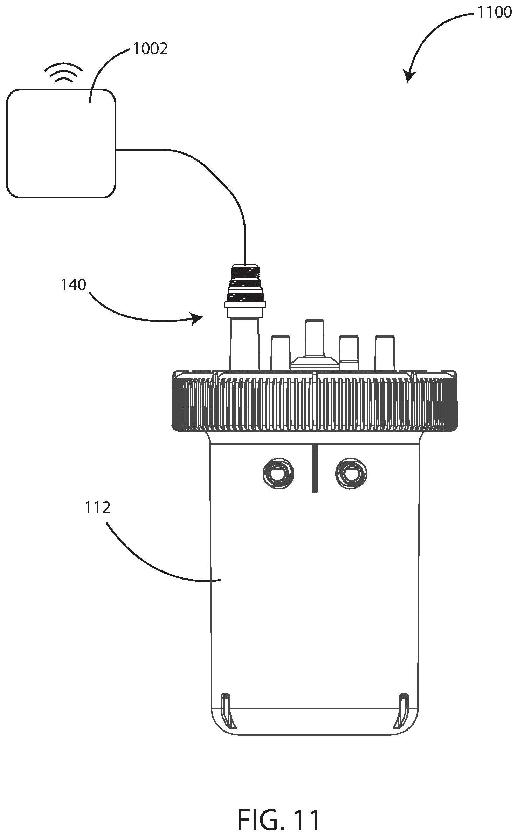

It will be appreciated that sensor systems herein can be used with many different types of bioreactors and other bioprocess equipment. As such, the bioreactor aspects shown with respect to FIGS. 1-3 herein are merely by way of non-limiting example. Referring now to FIG. 11, a schematic view is shown of another type of bioreactor system 1100 in accordance with various embodiments herein. In this example the bioreactor system 1100 includes a bioreactor housing 112 (which can be polymeric and disposable) and a multi-level liquid and foam sensor system 140 herein including a controller 1002. Specifically, the bioreactor housing 112 can include non-conductive walls and can define an internal volume. The multi-level liquid and foam sensor system 140 and/or components thereof such as a probe shaft can be configured to pass into the internal volume of the vessel and the multi-level liquid and foam sensor system 140 can be used to measure the presence and height of both liquid and foam within the bioreactor.

As previously described, the sensor system 140 can operate by detecting changes in electrical properties, such as conductance, resistance, capacitance, or impedance, between different vertically-spaced conductive probe wires. When the probe shaft is inserted into the internal volume of the bioreactor housing 112, the exposed end portions of the conductive probe wires come into contact with the contents inside the vessel. The controller 1002 can be configured to sense and analyze the electrical signals from these probe wires to determine whether a particular probe wire is in contact with air, foam, or liquid. By evaluating measured electrical properties at individual levels and/or by comparing the measured electrical properties at each probe position, the system can estimate the levels of liquid and foam, distinguish between liquid and foam phases, and/or detect the presence of foam above the liquid level. In some embodiments, the sensor system 140 can even detect levels of liquid and/or foam falling between probe positions by analyzing the variation in electrical property values measured at adjacent probe wires and interpolating the fluid or foam interface location between the discrete sensor positions. For example, when a transition in sensed electrical properties occurs between two adjacent probe wires, such as a marked change in an electrical property indicating a transition from air or foam to liquid, the controller can calculate a more precise interface location by applying an interpolation algorithm based on the relative differences in measured values. This approach can enable the sensor system to estimate the height of a liquid or foam level with greater resolution than the fixed spacing between probe wires, providing continuous monitoring capability within the region defined by the probe wire arrangement.

As described above, sensors herein can be used in conjunction with various types of bioprocess equipment, especially various types of disposable process equipment, including, but not limited to, disposable stirred tank bioreactors, disposable wave or rocking motion bioreactors, disposable bag bioreactors, disposable fixed bed and packed bed bioreactors, disposable fluidized bed bioreactors, disposable bubble column and airlift bioreactors, disposable photobioreactors, disposable media containers, disposable process tanks, disposable buffer tanks, disposable mixing vessels, disposable harvest and storage vessels, disposable transfer and transport containers, disposable tangential flow filtration (TFF) concentration vessels, and other single-use or limited-use liquid-handling vessels and containers commonly employed in bioprocessing operations.

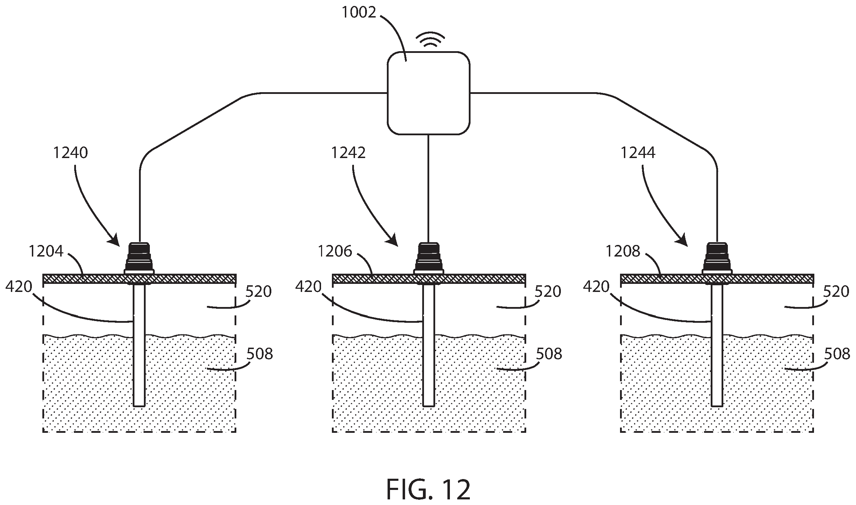

Referring now to FIG. 12, a schematic view of a bioprocess equipment system is shown in accordance with various embodiments herein. FIG. 12 shows a first sensor 1240 including a probe shaft 420 that passes through a first housing 1204 of a first piece of bioprocess equipment. FIG. 12 also shows a second sensor 1242 including a probe shaft 420 that passes through a second housing 1242 of a second piece of bioprocess equipment as well as a third sensor 1244 including a probe shaft 420 that passes through a third housing 1244 of a third piece of bioprocess equipment. In this example, all of the sensors 1240, 1242, and 1244 can in data communication with a single sensor system's controller 1002 (through wired or wireless means). However, it will be appreciated that the sensors could also be in data communication with separate controllers.

In some embodiments, data from the multiple sensors in multiple vessels can be integrated at the point of the controller 1002 or another piece of equipment in communication with the controller 1002 in order to provide process control over liquid level management, foam management, and associated bioprocess parameters across a plurality of bioprocess equipment units. By receiving and analyzing sensor data from each vessel, the controller 1002 can coordinate operations such as liquid transfers, dosing of antifoam agents, pump actuation, and other control actions, based on the monitored conditions within each vessel. This integration enables centralized or distributed process control, supporting improved consistency and reliability in the operation of connected bioprocess systems.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains. All publications and patent applications are herein incorporated by reference to the same extent as if each individual publication or patent application was specifically and individually indicated by reference.

As used herein, the recitation of numerical ranges by endpoints shall include all numbers subsumed within that range (e.g., 2 to 8 includes 2.1, 2.8, 5.3, 7, etc.).

The headings used herein are provided for consistency with suggestions under 37 CFR 1.77 or otherwise to provide organizational cues. These headings shall not be viewed to limit or characterize the invention(s) set out in any claims that may issue from this disclosure. As an example, although the headings refer to a "Field," such claims should not be limited by the language chosen under this heading to describe the so-called technical field. Further, a description of a technology in the "Background" is not an admission that technology is prior art to any invention(s) in this disclosure. Neither is the "Summary" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the spirit and scope herein.

## Claims

1. A multi-level liquid and foam sensor for bioprocess equipment comprising:
a probe shaft;
a plurality of conductive probe wires, wherein the plurality of conductive probe wires are arranged at different vertical levels along a height of the probe shaft; and
a controller;
wherein the controller is in electrical communication with the plurality of conductive probe wires;
wherein the controller is configured to
sense electrical properties of the plurality of conductive probe wires; and
estimate a liquid level within a piece of bioprocess equipment based on the sensed electrical properties.

2. The multi-level liquid and foam sensor for bioprocess equipment of claim 1, wherein the controller is configured to detect a height of liquid in the vessel using the sensed electrical properties of at least two conductive probe wires.

3. The multi-level liquid and foam sensor for bioprocess equipment of any of claims 1-2, wherein the controller is configured to detect the presence and/or a height of foam above the liquid level using the sensed electrical properties.

4. The multi-level liquid and foam sensor for bioprocess equipment of any of claims 1-3, the conductive probe wires comprising exposed end portions and/or wherein the plurality of conductive probe wires include an insulator coated over outside surfaces of the plurality of conductive probe wires other than at the exposed end portions thereof.

5. The multi-level liquid and foam sensor for bioprocess equipment of any of claims 1-4, wherein the length of the exposed end portions disposed outside of the probe shaft are at least 0.5 centimeters in length.

6. The multi-level liquid and foam sensor for bioprocess equipment of any of claims 1-5, wherein the sensed electrical properties include at least one of potential difference, conductance, resistance, capacitance, and impedance.

7. The multi-level liquid and foam sensor for bioprocess equipment of any of claims 1-6, wherein the plurality of conductive probe wires comprises from 2 to 15 conductive probe wires.

8. The multi-level liquid and foam sensor for bioprocess equipment of any of claims 1-7, wherein conductivity of a liquid within the piece of bioprocess equipment is determined based on electrical properties of a circuit including the at least two conductive probe wires disposed adjacent a bottom of the probe shaft.

9. The multi-level liquid and foam sensor for bioprocess equipment of any of claims 1-8, wherein the controller is further configured to adjust a sensitivity of foam detection based on the conductivity of the liquid within the piece of bioprocess equipment.

10. The multi-level liquid and foam sensor for bioprocess equipment of any of claims 1-9, wherein the controller is configured to calculate a liquid level, detect the presence of foam above the liquid level, detect the height of foam above the liquid level and/or detect the type of foam based on differing electrical properties between two conductive probe wires.

11. The multi-level liquid and foam sensor for bioprocess equipment of any of claims 1-10, further comprising a connection fitting, wherein the connection fitting is disposed adjacent a top of the probe shaft and allows the fluid and foam sensor to engage with a standardized port in a housing of the piece of bioprocess equipment, wherein the housing preferably comprises a non-conductive housing material.

12. The multi-level liquid and foam sensor for bioprocess equipment of any of claims 1-11, wherein the probe shaft is at least partially formed from a non-conductive material.

13. The multi-level liquid and foam sensor for bioprocess equipment of any of claims 1-12, wherein the probe shaft is disposable and the probe shaft is detachable from the controller.

14. The multi-level liquid and foam sensor for bioprocess equipment of any of claims 1-13, wherein the plurality of conductive probe wires include a stainless-steel conductor.

15. A piece of bioprocess equipment comprising:
a vessel with non-conductive walls defining an internal volume; and
a multi-level liquid and foam sensor comprising
a probe shaft configured to pass into the internal volume of the vessel;
a plurality of conductive probe wires, wherein the plurality of conductive probe wires are arranged at different vertical levels along a height of the probe shaft; and
a controller;
wherein the controller is in electrical communication with the plurality of conductive probe wires;
wherein the controller is configured to
sense electrical properties of the plurality of conductive probe wires; and
estimate a liquid level and/or detect the presence of foam above the liquid level within the piece of bioprocess equipment based on the sensed electrical properties.
